# EUROPEAN PATENT APPLICATION

(11) **EP 4 831 149 A1**
(43) Date of publication of application: **28.10.2026**
(21) Application number: 24906816.4
(22) Date of filing: 20.08.2024
(51) Int. Cl.: C07C 1/12, C07C 9/04

(54) **CONCENTRATION MEASUREMENT DEVICE, REACTION DEVICE, AND CONCENTRATION MEASUREMENT METHOD**

(30) Priority: 20.12.2023 JP 2023214500
(71) Applicant: Niterra Co., Ltd., Nagoya-shi, Aichi 461-0005 (JP)
(72) Inventor: MIZUNO, Akihito, Nagoya-shi, Aichi 461-0005 (JP); YABUHANA, Masaki, Nagoya-shi, Aichi 461-0005 (JP); IWASA, Shoto, Nagoya-shi, Aichi 461-0005 (JP)
(74) Representative: Grünecker Patent- und Rechtsanwälte PartG mbB
(86) International application number: PCT/JP2024/029527
(87) International publication number: WO 2025/134426

(57) **Abstract**

Provided are a concentration measurement device, a reaction device, and a concentration measurement method which make it possible to reduce the influence of pressure during concentration measurement for methane contained in a product gas. A concentration measurement device (20) includes a thermometer (23) for detecting a temperature of a product gas obtained by a methanation reaction between carbon dioxide and hydrogen, a pressure gauge (24) for detecting a pressure of the product gas, a flowmeter (22) for detecting a flow rate of the product gas or a flow rate of water contained in the product gas, and an processing unit (21) for calculating a concentration of methane, based on detection results of the thermometer, the pressure gauge, and the flowmeter.

## Description

### TECHNICAL FIELD

The present invention relates to a concentration measurement device for methane contained in a product gas obtained by a methanation reaction, a reaction device, and a concentration measurement method.

### BACKGROUND ART

There is a demand for concentration measurement for methane contained in a product gas obtained by a methanation reaction between carbon dioxide and hydrogen. In the conventional art disclosed in Patent Document 1, the concentration of carbon dioxide in a product gas is quantified to determine the conversion rate from carbon dioxide to methane.

### PRIOR ART DOCUMENT

### PATENT DOCUMENT

Patent Document 1: Japanese Patent Application Laid-Open (kokai) No. 2020-33280

### SUMMARY OF THE INVENTION

### PROBLEM TO BE SOLVED BY THE INVENTION

In the conventional art, when the conversion rate from carbon dioxide to methane is determined by measuring the carbon dioxide concentration of a product gas by using a concentration meter, the measurement is affected by the pressure of the product gas, leading to an inaccurate result. In a case of directly measuring the concentration of methane in the product gas by using a concentration meter, the measurement is affected by the pressure of the product gas as well, leading to an inaccurate result.

The present invention has been made to solve the above problem, and an object of the present invention is to provide a concentration measurement device, a reaction device, and a concentration measurement method which make it possible to reduce the influence of pressure during concentration measurement for methane contained in a product gas.

### MEANS FOR SOLVING THE PROBLEM

To attain the above object, a first aspect of the present invention is directed to a concentration measurement device for methane contained in a product gas obtained by a methanation reaction between carbon dioxide and hydrogen, the concentration measurement device including: a thermometer for detecting a temperature of the product gas; a pressure gauge for detecting a pressure of the product gas; a flowmeter for detecting a flow rate of the product gas; and an processing unit for calculating a concentration of the methane, based on detection results of the thermometer, the pressure gauge, and the flowmeter.

A second aspect is directed to the concentration measurement device according to the first aspect, wherein the processing unit determines a volumetric flow rate of saturated water vapor in the product gas, based on a saturated water vapor amount of the product gas at the temperature detected by the thermometer and the pressure of the product gas detected by the pressure gauge, determines a flow rate of carbon dioxide, based on the volumetric flow rate and the flow rate of the product gas detected by the flowmeter, and calculates the concentration of the methane contained in the product gas.

A third aspect is directed to a concentration measurement device for methane contained in a product gas obtained by a methanation reaction between carbon dioxide and hydrogen, the concentration measurement device including: a thermometer for detecting a temperature of the product gas; a pressure gauge for detecting a pressure of the product gas; a flowmeter for detecting a flow rate of the product gas; a water flow meter for detecting a flow rate of water formed through condensation of the product gas; and an processing unit for calculating a concentration of the methane, based on detection results of the thermometer, the pressure gauge, the flowmeter, and the water flow meter.

A fourth aspect is directed to the concentration measurement device according to the third aspect, wherein the processing unit determines a volumetric flow rate of saturated water vapor in the product gas, based on a saturated water vapor amount of the product gas at the temperature detected by the thermometer and the pressure of the product gas detected by the pressure gauge, determines a flow rate of carbon dioxide, based on the volumetric flow rate and the flow rate detected by the water flow meter, and calculates the concentration of the methane contained in the product gas, based on the flow rate of carbon dioxide and the flow rate of the product gas detected by the flowmeter.

A fifth aspect is directed to the concentration measurement device according to any one of the first to fourth aspects, wherein the product gas has an absolute pressure of 0.1 MPa or higher.

A sixth aspect is directed to a reaction device, including: the concentration measurement device according to any one of the first to fifth aspects; and a reactor in which a catalyst for promoting a methanation reaction between carbon dioxide and hydrogen is accommodated.

A seventh aspect is directed to the reaction device according to the sixth aspect, the reaction device including: a plurality of the reactors; a connecting passage which connects adjacent ones of the reactors and through which a product gas produced in a preceding stage reactor is sent to a subsequent stage reactor; and a separation device for separating water from the product gas in the connecting passage, wherein the concentration measurement device measures a concentration of methane contained in the product gas produced in a reactor at a stage preceding a final stage reactor.

An eighth aspect is directed to a concentration measurement method for methane contained in a product gas obtained by a methanation reaction between carbon dioxide and hydrogen, the method including: detecting a temperature of the product gas; detecting a pressure of the product gas; detecting a flow rate of the product gas or a flow rate of water contained in the product gas; and calculating a concentration of the methane, based on the temperature of the product gas, the pressure of the product gas, and the flow rate of the product gas or the flow rate of water contained in the product gas.

### ADVANTAGEOUS EFFECTS OF THE INVENTION

In the present invention, the concentration of methane is calculated based on the temperature of a product gas obtained by a methanation reaction between carbon dioxide and hydrogen, the pressure of the product gas, and the flow rate of the product gas or the flow rate of water contained in the product gas without using a concentration meter, whereby the influence of the pressure of the product gas can be reduced. In addition, the concentration meter can be omitted.

### BRIEF DESCRIPTION OF THE DRAWINGS

[FIG. 1] Block diagram showing a reaction device according to a first embodiment.
[FIG. 2] Block diagram showing a reaction device according to a second embodiment.

### MODES FOR CARRYING OUT THE INVENTION

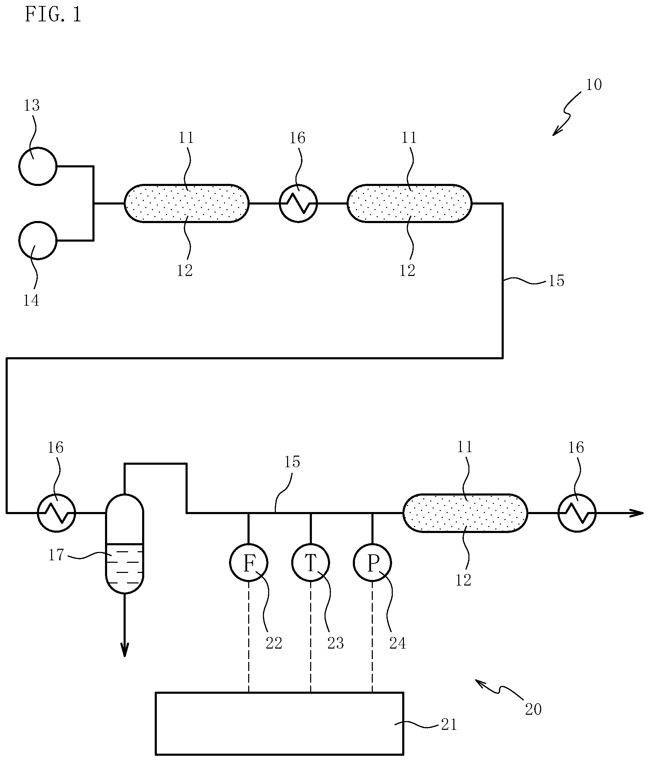

Hereinafter, preferred embodiments of the present invention will be described with reference to the accompanying drawings. FIG. 1 is a block diagram of a reaction device 10 according to a first embodiment. The reaction device 10 includes a reactor 11 in which a catalyst 12 is accommodated, a carbon dioxide introduction unit 13 that introduces carbon dioxide into the reactor 11, and a hydrogen introduction unit 14 that introduces hydrogen into the reactor 11. A methanation reaction, represented by the chemical reaction equation of CO₂ + 4H₂ → CH₄ + 2H₂O, proceeds in the reactor 11 into which a feed gas containing carbon dioxide and hydrogen is introduced. The reaction device 10 includes a concentration measurement device 20 that measures the concentration of methane contained in a product gas obtained by the methanation reaction.

The catalyst 12 used may be any catalyst suitable for the methanation reaction without limitation. Examples of the catalyst 12 include powder, pellets, and a porous structure in which particles are supported on a carrier. Examples of the carrier include powder, pellets, and a porous structure of an oxide containing one or more of alumina, silica, magnesia, titania, zirconia, niobia, silica-alumina, zeolite, and calcium phosphate. The porous structure has gas permeability that allows the feed gas to pass therethrough. The feed gas passes through gaps in the powder or in the pellets. Examples of the particles supported on the carrier include metals such as Ni.

The reaction device 10 includes a plurality of reactors 11 (three reactors for the present embodiment) and connecting passages 15 connecting the adjacent reactors 11. The connecting passages 15 each allow the product gas produced by the methanation reaction in a preceding stage reactor 11 to be sent to a subsequent stage reactor 11. Since the catalyst 12 is not disposed in the connecting passages 15, the methanation reaction does not proceed when the product gas passes through the connecting passages 15. The number of reactors 11 is not limited to three and may be any number not smaller than two.

Coolers 16 are disposed in the connecting passages 15. A cooler 16 is also disposed downstream of the final reactor 11. The coolers 16 used may be any cooler capable of cooling the product gas, such as a heat exchanger, without limitation. The coolers 16 cool the product gas produced in the preceding stage reactors 11, and the connecting passages 15 each allow the cooled product gas to be sent to the subsequent stage reactors 11. The coolers 16 cool the product gas, for example, to the lowest temperature required to start the methanation reaction. Since the methanation reaction is an exothermal reaction, a reverse reaction becomes dominant if the temperature of the surface of the catalyst 12 becomes high, resulting in reduction in the conversion rate to methane. The coolers 16 are provided in order to reduce the reverse reaction and increase the conversion rate to methane.

A separation device 17 for separating water from the product gas is disposed downstream of a cooler 16 in the connecting passages 15. The cooler 16 disposed upstream of the separation device 17 cools the product gas to the dew point, and the separation device 17 separates water condensed by the cooler 16. When the amount of water contained in the product gas is decreased by the separation device 17, the speed of the forward reaction becomes higher than the speed of the reverse reaction of the methanation reaction in the reactor 11 downstream of the separation device 17, making it possible to increase the conversion rate to methane.

The concentration measurement device 20 is a device for measuring the concentration of methane contained in the product gas. The concentration measurement device 20 measures the concentration of methane contained in the product gas flowing downstream of a reactor 11 at a stage preceding the final stage reactor 11. The absolute pressure of the product gas measured by the concentration measurement device 20 is, for example, 0.1 MPa or higher. The concentration of methane contained in the product gas in the final stage reactor 11 can be measured by using a concentration meter after the pressure of the product gas is reduced to atmospheric pressure.

The concentration measurement device 20 includes an processing unit 21, a flowmeter 22 for measuring the flow rate of the product gas, a thermometer 23 for detecting the temperature of the product gas, and a pressure gauge 24 for detecting the pressure of the product gas. The flowmeter 22, the thermometer 23, and the pressure gauge 24 input respective detection results into the processing unit 21.

The processing unit 21 includes a CPU, a ROM, a RAM and a backup RAM (which are not shown). The ROM is a nonvolatile memory storing a program to be executed by the CPU. The CPU executes arithmetic processing on the basis of the program stored in the ROM. The RAM is a memory for temporarily storing an arithmetic result of the CPU, data inputted from each sensor, or the like. The backup RAM is a nonvolatile memory for storing data or the like that should be saved.

The chemical reaction equation of the methanation reaction is CO₂ + 4H₂ → CH₄ + 2H₂O. Thus, among flow rates of the product gas after the reaction, if the flow rate of CO₂ is x [L/min], the flow rate of H₂ is 4x [L/min]. When taking the conversion rate from CO₂ to CH₄ into consideration, the flow rate of CH₄ contained in the product gas is 1 - x [L/min]. The product gas also contains saturated water vapor, and thus, if the saturated water vapor amount is α [L/min], the flow rate F of the product gas detected by the flowmeter 22 is F = x + 4x + (1-x) + α = 4x + 1 + α [L/min].

The saturated water vapor amount [g/m³] is a value determined by the temperature of the product gas detected by the thermometer 23. The ROM of the processing unit 21 stores the saturated water vapor amount [g/m³] for each temperature. The processing unit 21 converts a fluid density [g/m³] into a volumetric flow rate [L/min], based on the expression Q = A·V = A·(2q/P)^{1/2}, where Q represents the volumetric flow rate = the saturated water vapor amount [L/min], A represents the cross-sectional area of the connecting passages 15, V represents the flow velocity, q represents the pressure of the product gas detected by the pressure gauge 24, and P represents the fluid density = the saturated water vapor amount [g/m³]. A is a known constant in the expression Q = A·(2q/P)^{1/2}. Thus, the processing unit 21 calculates the amount α [L/min] of saturated water vapor contained in the product gas by substituting the pressure of the product gas detected by the pressure gauge 24 and the saturated water vapor amount [g/m³] which is determined by the temperature detected by the thermometer 23.

The processing unit 21 obtains the flow rate F [L/min] of the product gas detected by the flowmeter 22 and calculates x by substituting the saturated water vapor amount α [L/min] into the algebraic expression of F = 4x + 1 + α. The flow rate of CH₄ contained in the product gas is 1 - x [L/min]. Thus, the concentration [vol%] of CH₄ contained in the product gas is (1-x)/F. The processing unit 21 calculates the concentration [vol%] of CH₄ contained in the product gas by substituting x and F into this algebraic expression.

Since the concentration measurement device 20 measures the concentration of methane contained in the product gas without using a concentration meter, the measurement result of which is easily affected by the pressure, it is possible to reduce the influence of pressure during concentration measurement. As a result, it is possible to measure the concentration of methane contained in a high-pressure product gas flowing through the connecting passages 15 connecting the adjacent reactors 11. By disposing the concentration measurement device 20 in each of the connecting passages 15, the methane concentration can be measured for each of the reactors 11. Thus, it is possible to detect the state of deterioration of the catalyst 12 accommodated in the reactors 11 by comparing a methane concentration designed for each reaction stage with the actual methane concentration. Since a deteriorated catalyst 12 can be replaced appropriately, the maintenance of the reaction device 10 becomes easy. Since the concentration meter for detecting the concentration of a component of the product gas can be omitted, it is possible to eliminate maintenance of the concentration meter, which is part of the maintenance of the reaction device.

By disposing the concentration measurement device 20 in each of the connecting passages 15, the methane concentration can be measured for each of the reactors 11, thus making it possible to detect an abnormality in the concentration of carbon dioxide introduced into the reactor 11 by the carbon dioxide introduction unit 13, by comparing the methane concentration of the product gas in the foremost stage reactor 11 with the methane concentration designed for each reaction stage. As a result, it is possible to detect an abnormality in the feed gas.

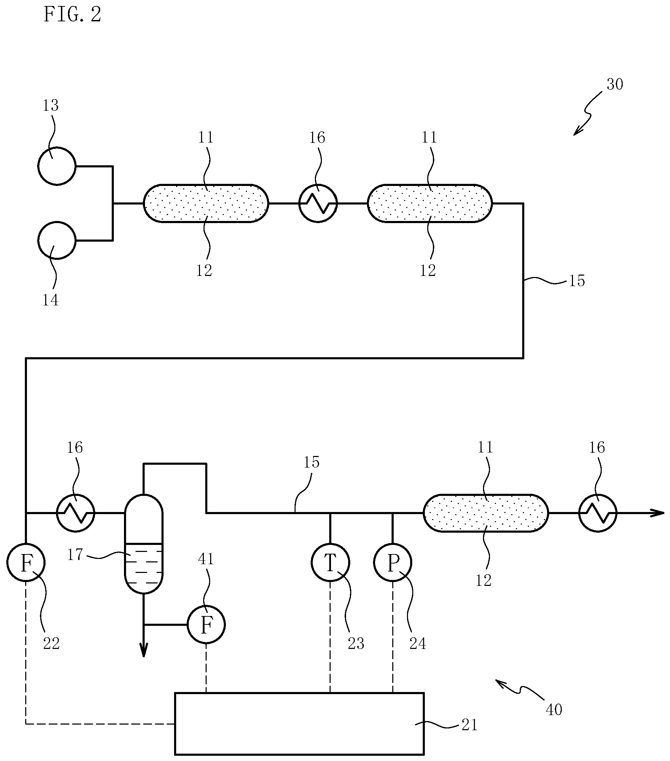

A second embodiment of the present invention will be described with reference to FIG. 2. In the first embodiment, a case of determining the methane concentration of the product gas by detecting the flow rate of the product gas has been described. On the other hand, in the second embodiment, a case of determining the methane concentration of the product gas by detecting the flow rate of water formed through condensation of water vapor contained in the product gas will be described. In the second embodiment, the same parts as those described in the first embodiment are denoted by the same reference characters and description thereof will not be repeated below.

FIG. 2 is a block diagram of a reaction device 30 according to the second embodiment. The reaction device 30 includes a concentration measurement device 40. The concentration measurement device 40 measures the concentration of methane contained in the product gas flowing downstream of a reactor 11 at a stage preceding the final stage reactor 11.

The concentration measurement device 40 includes an processing unit 21, a flowmeter 22, a thermometer 23, a pressure gauge 24, and a water flow meter 41 (flowmeter) for detecting a flow rate of water separated by a separation device 17. The flowmeter 22 detects the flow rate F of the product gas upstream of the separation device 17. The thermometer 23 and the pressure gauge 24 respectively detect the temperature and the pressure of the product gas downstream of the separation device 17. The water flow meter 41, the flowmeter 22, the thermometer 23, and the pressure gauge 24 input respective detection results into the processing unit 21.

The chemical reaction equation of the methanation reaction is CO₂ + 4H₂ → CH₄ + 2H₂O. Thus, among flow rates of the product gas after the reaction, if the flow rate of CO₂ is x [L/min], the flow rate of H₂ is 4x [L/min]. When taking the conversion rate from CO₂ to CH₄ into consideration, the flow rate of CH₄ contained in the product gas is 1 - x [L/min] and the flow rate of H₂O (gas) is 2(1 - x) [L/min].

If the flow rate detected by the water flow meter 41, i.e., the flow rate of H₂O (liquid) separated by the separation device 17, is β [L/min], β [L/min] = β [g/mol] = β/18 [mol/min]. Thus, according to Avogadro's law, the flow rate of H₂O (gas) separated by the separation device 17 is β/18 [mol/min]·22.4 [L/mol] = 22.4β/18 [L/min]. The product gas also contains saturated water vapor, and thus, if the saturated water vapor amount of the product gas flowing downstream of the separation device 17 is α [L/min], the flow rate of H₂O (gas) contained in the product gas flowing upstream of the separation device 17 is 22.4β/18 + α [L/min].

When focusing on the flow rate [L/min] of H₂O (gas) contained in the product gas, 2(1 - x) = 22.4β/18 + α is satisfied. As in the first embodiment, the processing unit 21 calculates the amount α [L/min] of saturated water vapor contained in the product gas, from the pressure of the product gas detected by the pressure gauge 24 and the saturated water vapor amount [g/m³] determined by the temperature detected by the thermometer 23. The processing unit 21 calculates x by substituting the saturated water vapor amount α and the flow rate β detected by the water flow meter 41 into the expression of 2(1 - x) = 22.4β/18 + α.

The processing unit 21 obtains the flow rate F [L/min] of the product gas detected by the flowmeter 22. Since the flow rate of CH₄ contained in the product gas is 1 - x [L/min], the concentration [vol%] of CH₄ contained in the product gas is (1 - x)/F. The processing unit 21 calculates the concentration [vol%] of CH₄ contained in the product gas by substituting x and F into this algebraic expression.

The concentration measurement device 40 and the reaction device 30 including the concentration measurement device 40 can achieve the same effect as in the concentration measurement device 20 and the reaction device 10 according to the first embodiment.

Although the present invention has been described with reference to the embodiments, the present invention is not limited to the above-described embodiments in any way. It can be easily understood that various modifications can be devised without departing from the gist of the present invention.

In the first embodiment, the methane concentration of the product gas is calculated by detecting the flow rate, the temperature, and the pressure of the product gas flowing downstream of the coolers 16 in the connecting passages 15, but the present invention is not necessarily limited thereto. As a matter of course, the methane concentration of the product gas can be calculated by detecting the flow rate, the temperature, and the pressure of the product gas flowing upstream of the coolers 16 in the connecting passages 15.

In the above embodiments, a case where the separation device 17 is disposed in one of the three connecting passages 15 has been described, but the present invention is not necessarily limited thereto. As a matter of course, separation devices 17 can be respectively disposed in three connecting passages 15, or separation devices 17 can be disposed in two connecting passages 15 among three connecting passages 15. As a matter of course, a plurality of separation devices 17 can be disposed in one connecting passage 15.

In the above embodiments, the reaction devices 10, 30 which are each provided with the separation device 17 for separating water condensed by the coolers 16 have been described, but the separation device 17 is not limited thereto. The separation device 17 may be a device that uses a desiccant such as slaked lime, an adsorbent such as activated carbon or zeolite, a water separation membrane, or the like to separate water vapor without condensing water vapor by the coolers 16.

In the above embodiments, a case where the coolers 16 cool the product gas to the lowest temperature required to start the methanation reaction has been described, but the present invention is not necessarily limited thereto. The temperature to which the product gas is cooled by the coolers 16 may be any temperature less than the temperature at which the methanation reaction reaches equilibrium.

Although not described in the above embodiments, as a matter of course, it is possible to provide a heater to the connecting passages 15 or the reactors 11 so as to heat a gas introduced into the reactors 11 to a temperature higher than the temperature at which the methanation reaction starts. Examples of the heater include one that heats the product gas by heating a heat source by means of gas combustion heat or electricity, one that utilizes induction heating, and a heat exchanger.

### DESCRIPTION OF REFERENCE NUMERALS

10,30 reaction device
11 reactor
12 catalyst
15 connecting passage
17 separation device
20,40 concentration measurement device
21 processing unit
22 flowmeter
23 thermometer
24 pressure gauge
41 water flow meter

## Claims

1. A concentration measurement device for methane contained in a product gas obtained by a methanation reaction between carbon dioxide and hydrogen, the concentration measurement device comprising:
a thermometer for detecting a temperature of the product gas;
a pressure gauge for detecting a pressure of the product gas;
a flowmeter for detecting a flow rate of the product gas; and
an processing unit for calculating a concentration of the methane, based on detection results of the thermometer, the pressure gauge, and the flowmeter.

2. A concentration measurement device for methane contained in a product gas obtained by a methanation reaction between carbon dioxide and hydrogen, the concentration measurement device comprising:
a thermometer for detecting a temperature of the product gas;
a pressure gauge for detecting a pressure of the product gas;
a flowmeter for detecting a flow rate of the product gas;
a water flow meter for detecting a flow rate of water formed through condensation of the product gas; and
an processing unit for calculating a concentration of the methane, based on detection results of the thermometer, the pressure gauge, the flowmeter, and the water flow meter.

3. The concentration measurement device according to claim 1 or 2, wherein the product gas has an absolute pressure of 0.1 MPa or higher.

4. A reaction device comprising:
the concentration measurement device according to claim 1 or 2; and
a reactor in which a catalyst for promoting a methanation reaction between carbon dioxide and hydrogen is accommodated.

5. The reaction device according to claim 4,
comprising:
a plurality of the reactors;
a connecting passage which connects adjacent ones of the reactors and through which a product gas produced in a preceding stage reactor is sent to a subsequent stage reactor; and
a separation device for separating water from the product gas in the connecting passage, wherein
the concentration measurement device measures a concentration of methane contained in the product gas produced in a reactor at a stage preceding a final stage reactor.

6. A concentration measurement method for methane contained in a product gas obtained by a methanation reaction between carbon dioxide and hydrogen, the concentration measurement method comprising:
detecting a temperature of the product gas;
detecting a pressure of the product gas;
detecting a flow rate of the product gas or a flow rate of water contained in the product gas; and
calculating a concentration of the methane, based on the temperature of the product gas, the pressure of the product gas, and the flow rate of the product gas or the flow rate of water contained in the product gas.
